# FASCICULE DE BREVET EUROPEEN

(11) **EP 0 452 231 B1**
(45) Date de publication et mention de la délivrance du brevet: **06.07.1994**
(21) Numéro de dépôt: 91420117.3
(22) Date de dépôt: 08.04.1991
(51) Int. Cl.: A61L 11/00

(54) **Procédé de stérilisation de déchets contaminés et dispositif pour sa mise en oeuvre**
Verfahren zur Sterilisation von kontaminierten Abfällen und Vorrichtung zu seiner Ausführung
Method for sterilization of contaminated waste and device for carrying-out thereof

(30) Priorité: 09.04.1990 FR 9004960
(43) Date de publication de la demande: 16.10.1991
(73) Titulaire: Roux, Jean, Louis, Emile, F-69006 Lyon (FR)
(72) Inventeur: Roux, Jean, Louis, Emile, F-69006 Lyon (FR)
(74) Mandataire: Maureau, Philippe

(56) Documents cités:
- FR-A- 2 078 203

## Description

La présente invention a pour objet un procédé de stérilisation de déchets contaminés et un dispositif pour sa mise en oeuvre.

On entend par déchets contaminés, tous déchets biologiquement contaminés (c'est-à-dire contaminés par une source autre que celle provenant de radiations nucléaires) qui, en raison de leur nature, doivent être conditionnés dans des contenants spéciaux pour éviter leur mélange avec les autres déchets dits banaux et évacués en vue de leur traitement et de leur élimination dans un délai maximal de 48 heures.

C'est ainsi que, les déchets hospitaliers contaminés, ou encore désignés par la législation par le terme de "spécifiques" et classés en deuxième catégorie (par différence avec les déchets banaux urbains classés en première catégorie) sont constitués par tout objet médical ou non qui a été en contact avec un malade atteint d'une maladie infectieuse grave, telle que par exemple le Sida, l'Hépatite B, ou autre, et ils doivent subir ces traitements.

Pour ce faire, un procédé d'élimination de ces déchets consiste à les enfouir dans des décharges contrôlées après qu'ils aient subi un traitement préalable. Ils sont ainsi empilés sur différentes couches successives recouvertes chacune par des agents chimiques désinfectants et dégradants. Ce procédé, on le conçoit, présente les inconvénients de dégrader l'environnement naturel du site sur lequel il est mis en oeuvre ainsi que de polluer les nappes phréatiques par infiltration.

Pour remédier à ces inconvénients, il est connu d'avoir recours à l'incinération de ces déchets à l'intérieur de fours dont la température s'élève à plus de 1100°C suivant les normes imposées.

Or, il s'avère que généralement, ces fours d'incinération ne sont pas conformes à la règlementation, et notamment, qu'ils ne sont pas adaptés pour atteindre de telles températures. De plus, les résidus de combustion (cendres, imbrûlés, fumées) ne subissent pas toujours un retraitement spécifique et de ce fait sont des facteurs importants de pollution et de contamination. Enfin, les fumées de combustion dégagées, lors de l'incinération de ces déchets, ne sont souvent pas filtrées, ce qui est une autre source de nuisance et de pollution atmosphérique.

Afin d'éviter ces différentes pollutions, et les dangers de contamination qu'elles engendrent, il a été mis en oeuvre des unités de traitement centralisées de ces déchets. Toutefois, en raison du coût important de leur fonctionnement et de leur construction, sur des sites éloignés des sources de ces déchets, de telles unités ne sont restées qu'au stade de petites unités expérimentales.

Or, le développement des produits médicaux à usage "unique" ainsi que celui des maladies infectieuses ont entraîné une augmentation du volume des déchets médicaux et notamment de déchets spécifiques. C'est la raison pour laquelle, il a été développé un procédé de traitement de ces déchets dans des unités mobiles en leur faisant subir un traitement thermique par passage sur une succession de générateurs de micro-ondes.

Toutefois, un tel procédé, bien que présentant l'avantage de pouvoir être utilisé sur différents sites, présente l'inconvénient d'une part d'être de maintenance complexe, et d'autre part de ne permettre qu'une réduction du nombre de germes par gramme, que ces déchets contiennent, à un seuil compris entre 10⁻² et 10⁻³ c'est-à-dire correspondant à leur décontamination.

La présente invention a pour objet de remédier à ces inconvénients en fournissant un procédé de stérilisation de déchets contaminés qui permet une diminution à un seuil inférieur à 10⁻⁶ du nombre de germes par gramme qu'ils contiennent. De plus, un dispositif pour la mise en oeuvre de ce procédé a pour but d'éviter toute contamination extérieure.

A cet effet, ce procédé de stérilisation de déchets contaminés consiste à broyer les déchets contaminés pour obtenir un mélange de granulométrie homogène, à amener le mélange broyé sur un transporteur, à niveler le mélange pour obtenir une couche d'épaisseur prédéterminée sur le transporteur et à déplacer le mélange par l'intermédiaire du transporteur à une vitesse déterminée, pour réaliser l'irradiation de la couche de déchets par le champ d'un faisceau d'électrons.

Ce procédé de stérilisation permet grâce d'une part au broyage de ces déchets en un mélange de particules de petites dimensions de granulométrie comprises entre 1 et plusieurs cm et d'autre part au nivellement de ce mélange de déchets contaminés en une couche d'épaisseur régulière comprise entre 1 et plusieurs cm d'obtenir une bonne stérilisation de ces déchets sous le champ d'un faisceau d'électrons, du fait que l'on maîtrise la dose d'irradiation, connaissant le débit d'amenée des déchets broyés, l'épaisseur de la couche de déchets et les caractéristiques du faisceau d'électrons.

Cette stérilisation des déchets permet ensuite leur traitement comme des déchets dits banaux.

Selon une caractéristique intéressante de l'invention, ce procédé consiste à déposer ce mélange de déchets contaminés à l'extrémité amont d'un transporteur réalisant l'amenée des déchets sous le champ d'un faisceau d'électrons et permettant leur évacuation à partir de son extrémité aval vers l'extérieur.

Avantageusement, ce procédé consiste à réaliser l'amenée de manière discontinue des déchets à l'intérieur d'un contenant étanche, à faire chuter, par gravité, les déchets dans un broyeur et à évacuer, par gravité, en continu et de façon régulée, ce mélange de déchets contaminés sur l'extrémité amont du transporteur.

Selon une caractéristique intéressante de l'invention, un dispositif pour la mise en oeuvre du procédé comprend une enceinte de traitement à l'intérieur de laquelle se trouve un broyeur pour réduire les déchets contaminés en un mélange de granulométrie homogène, un transporteur, un moyen de nivellement pour obtenir une couche d'épaisseur régulière du mélange de déchets et un accélérateur d'électrons pour irradier et stériliser la couche de déchets contaminés.

Avantageusement, un transporteur à bande est destiné pour faire défiler depuis son extrémité amont jusqu'à son extrémité aval la couche de déchets contaminés.

Ce transporteur dont la largeur est sensiblement identique à celui du champ du faisceau d'électrons permet un convoyage régulier de ces déchets. De plus, il comporte des rebords latéraux pour éviter toute chute non désirée de particules de ce mélange de déchets contaminés.

Selon une caractéristique intéressante de l'invention, l'accélérateur d'électrons comprend une cuve à l'intérieur de laquelle règne une pression de gaz isolant électrique ainsi qu'un tube accélérateur générant un faisceau d'électrons d'énergie comprise entre 1 et 3 Méga ElectronVolt, et un dispositif de balayage comportant au moins un aimant générant un champ magnétique et se terminant par une fenêtre séparant la zone de vide de l'atmosphère.

Cet accélérateur d'électrons génère un faisceau d'électrons d'énergie comprise entre 1 et 3 Méga ElectronVolt qui permet une irradiation et une stérilisation homogène de la couche de mélange de déchets par une dose d'irradiation comprise entre 10 et 50 kilo.Gray.

Avantageusement, l'enceinte de traitement est agencée pour être disposée sur un véhicule.

Cela permet la mise en place sur différents sites de ce dispositif. Il est ainsi possible de traiter sur place, les déchets contaminés provenant d'établissements hospitaliers de petite et moyenne importance ce qui en augmente les capacités d'utilisation.

De toute façon, l'invention sera bien comprise, à l'aide de la description qui suit, en référence au dessin schématique annexé, représentant à titre d'exemple non limitatif, une forme de réalisation de ce dispositif.

Figure 1 est une vue très schématique, en coupe longitudinale, de l'ensemble du dispositif.

Figure 2 est une vue, en coupe transversale, représentant plus particulièrement l'accélérateur d'électrons.

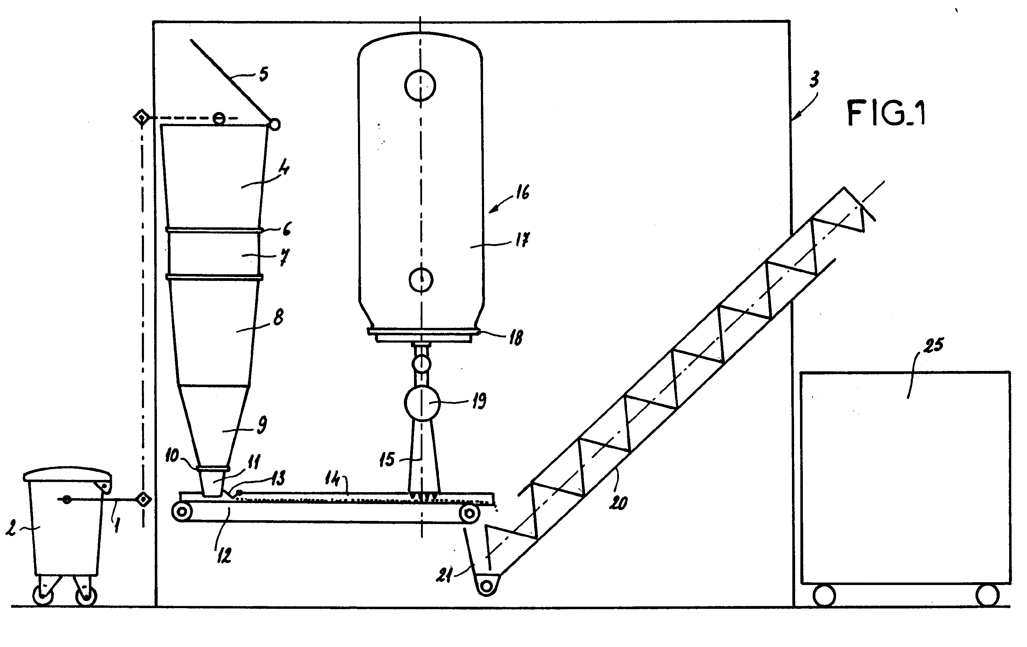

Ce dispositif comprend, comme mieux illustré à la figure 1, un bras articulé 1, qui permet la préhension et l'introduction d'un conteneur 1 à l'intérieur d'une enceinte 3. Dans ce conteneur 2, se trouvent des déchets biologiquement contaminés provenant essentiellement d'établissements hospitaliers. Ces déchets, en raison de leur spécificité et de leur origine, sont conditionnés dans des conteneurs spéciaux et doivent être éliminés sous 48 heures.

Ce bras articulé 1 est agencé pour permettre, l'élévation du conteneur 2 jusqu'à une ouverture d'entrée, non représentée, ménagée dans l'extrémité supérieure de l'enceinte 3 et le déchargement de ce conteneur 2, après son ouverture et sa rotation de 90°, dans une trémie étanche 4. Cette trémie 4 est fermée par un couvercle 5 qui s'ouvre lors de l'introduction du conteneur 2 à l'intérieur de l'enceinte 3 et qui se referme après la sortie de ce conteneur 2 hors de cette enceinte 3.

L'extrémité inférieure de cette trémie 4 comporte un orifice plus ou moins obturable par une trappe 6. Les déchets contaminés introduits de façon discontinue, à l'intérieur de cette trémie 5, tombent par gravité de manière continue et selon un débit régulé, et débouchent dans un broyeur, non représenté, situé sous cette ouverture à l'intérieur d'une chambre de broyage 7. Ce broyeur réduit en un mélange de granulométrie homogène constitué de petites particules de dimensions comprises par exemple entre 1 et 3 cm et de préférence entre 1 et 1,5 cm, ces déchets hétérogènes constitués de matières textiles et plastiques diverses, de métal, de verre, de papier... Cette chambre 7 de broyage débouche dans un contenant 8 où tombe, par gravité, le mélange de déchets contaminés et où il est stocké temporairement. L'extrémité inférieure de ce contenant 8, en forme de cône 9, comporte un orifice 10, plus ou moins obturable par une trappe 11 située au dessus de l'extrémité amont du transporteur à bandes.

Le mélange de déchets contaminés tombe, par gravité, sur le transporteur à bande 12, selon un débit régulé par la trappe 11. Une râcle 13 située en aval de cet orifice 10, au-dessus du transporteur, à une distance prédéterminée de celui-ci, permet de niveler ce mélange de déchets contaminés en une couche d'épaisseur régulière et quasi constante comprise entre par exemple 1 et 3 cm et de préférence entre 0,5 et 1,5 cm.

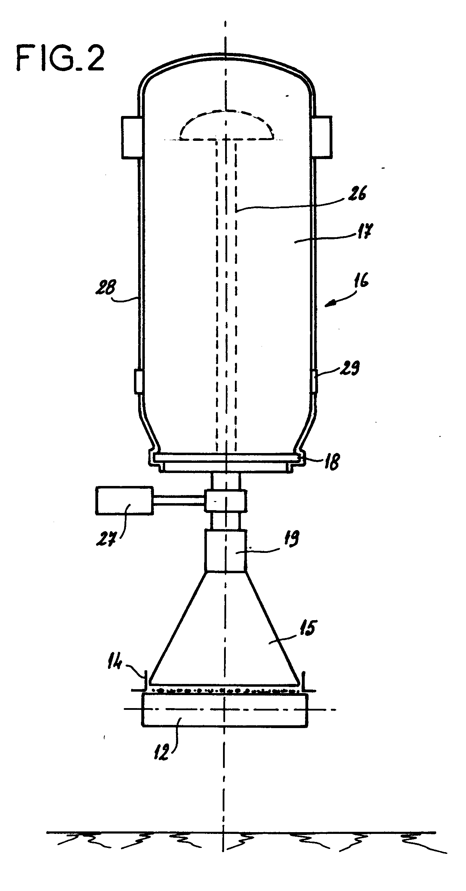

Le transporteur à bande 12, de longueur comprise entre 2 et 4 m est entraîné par des moyens connus non représentés. Il comporte, sur chaque côté, un rebord latéral 14 afin d'éviter toute chute de déchets contaminés et possède une largeur sensiblement identique à celle du champ du faisceau d'électrons 15 émis par un dispositif de balayage 18 associé à un accélérateur d'électrons 16, tel que représenté à la figure 2.

Cet accélérateur d'électrons 16, d'un type connu, comprend une cuve 17 à l'intérieur de laquelle règne une pression d'un gaz isolant électrique et où se trouve un tube accélérateur 26, sous un vide poussé généré par un dispositif de pompage 27 d'un type connu. Ce vide permet de favoriser le trajet des faisceaux d'électrons, d'énergie comprise entre 1 et 3 Méga ElectronVolt et de préférence de 2,2 Méga ElectronVolt depuis ce tube accélérateur 26 jusqu'à un dispositif de balayage 18.

Le dispositif de balayage 18 comprend un électro-aimant qui génère un champ magnétique qui permet la déviation du faisceau d'électrons et se termine par une fenêtre qui sépare la zone de vide poussé de l'atmosphère. Le champ du faisceau d'électrons 15, traverse facilement cette fenêtre et il irradie et stérilise, pendant une durée d'environ une à deux minutes, la couche de mélange de déchets contaminés disposée sur le transporteur à bande lors de son passage sous ce champ 15. Cette durée d'irradiation, pour obtenir une dose d'irradiation suffisante, peut varier en fonction de la nature des déchets à stériliser, par simple réglage de la vitesse de défilement du transporteur à bande 12. La dose d'irradiation délivrée est comprise entre 10 et 50 Kilo.Gray et est suffisante pour stériliser ces déchets contaminés, c'est-à-dire obtenir un nombre de germes par gramme qu'ils contiennent inférieurs à 10⁻⁶.

Cette irradiation se fait par balayage par un champ d'un faisceau d'électrons 15 ou cône de balayage d'une largeur comprise entre 0,6 et 1 m dont l'axe du champ du faisceau 15 est dirigé perpendiculairement à la direction de déplacement du transporteur à bande 12. La distance séparant l'extrémité du champ du faisceau 15 de celle du transporteur 12 est définie et réglée en fonction de la nature des produits à stérilier.

Pour éviter tout échauffement de l'accélérateur d'électrons 16, est prévu un circuit fermé de refroidissement dans lequel circule un fluide, tel que de l'eau.

Il est ainsi tout à fait envisageable, après un réglage spécifique et adapté, que l'axe du champ du faisceau d'électrons 15 forme un angle avec la perpendiculaire à la direction de déplacement du transporteur à bande 12, ce qui permet d'obtenir une surface de la couche de mélange de déchets contaminés irradiée plus importante.

Cette couche de mélange de déchets irradiés est stérilisée et convoyée jusqu'à l'extrémité aval du transporteur à bandes 12 disposée au dessus d'une goulotte de réception 21 disposée à l'extrémité amont d'un transporteur à vis 20. Cette couche de déchets stérilisés tombe par gravité à l'intérieur de cette goulotte de réception 21 et ces déchets stérilisés sont convoyés et évacués à l'extérieur de l'enceinte 3. Ils sont déversés dans un contenant de réception 25 pour être ensuite retraités soit par incinération, soit par dépôt dans une décharge comme des déchets dits banaux.

L'ensemble de ce dispositif est régulé et commandé à partir, par exemple d'un automate programmable, qui contrôle l'introduction des déchets contaminés de manière discontinue à l'intérieur de la trémie étanche 4 dans l'enceinte 3, ainsi que d'une part l'ouverture plus ou moins importante des trappes 6, 11 et d'autre part la vitesse d'avancement du transporteur à bandes 12 et la durée et la dose d'irradiation de la couche de mélange de déchets contaminés.

Pour permettre un contrôle manuel, depuis l'extérieur de l'enceinte 3, cet automate est connecté par exemple à un coffret de commande à distance portable comportant des voyants lumineux qui indiquent le bon fonctionnement des divers organes électriques et/ou électromécaniques du dispositif.

La protection contre les radiations émises par l'accélérateur d'électrons 16, est assurée par du béton, une épaisseur suffisante pour tenir compte des effets primaires et secondaires de ces radiations. C'est ainsi qu'afin d'éviter une émission de rayons X à l'extérieur de l'enceinte 3, celle-ci est réalisée par l'empilage de moellons de béton d'épaisseur suffisante. Cette enceinte 3 peut, lorsque cela est possible, tenir compte des configurations du site sur lequel le dispositif est mis en oeuvre.

L'indication de la présence de ce dispositif et de son fonctionnement, pour éviter tout risque de contamination de personnes, est conforme aux normes en vigueur. Notamment, elle comprend une indication du taux de radiation qui règne en divers points plus ou moins éloignés de la zone de fonctionnement de ce dispositif, ainsi qu'une signalisation lumineuse à partir de panneaux interdisant l'accès à toute personne non autorisée de cette zone.

Dans une autre forme de réalisation de ce dispositif, l'enceinte 3 est agencée pour être disposée sur un véhicule adapté tel qu'un plateau de semi-remorque. Cela permet de pouvoir stériliser les déchets contaminés sur différents sites et notamment dans les établissements hospitaliers ou similaires de petite ou moyenne importance. On conçoit que cela permet d'augmenter considérablement les capacités d'utilisation d'un tel dispositif.

Pour éviter toute perte de place à l'intérieur de l'enceinte 3, disposée sur un véhicule, l'accélérateur d'électrons 16 est déplaçable entre deux positions autour d'un axe de rotation 29. Une première position, qui est celle de fonctionnement, où il est disposé tel que représenté aux figures 1 et 2, verticalement perpendiculairement à la direction d'avancement du transporteur à bande 12, et une seconde position, obtenue après une rotation de 90°, qui est celle de transport, où il est disposé horizontalement parallèlement à la direction d'avancement du transporteur à bandes 12.

De même, pour faciliter la mise en place ce l'ensemble des éléments du dispositif à l'intérieur de l'enceinte 3, le transporteur à vis 20 est constitué d'éléments modulaires repliables que l'on assemble, en fonction de la configuration du site où ce dispositif est mis en oeuvre. Cet assemblage, permet de réaliser une longueur suffisante de vis pour obtenir le déchargement des déchets stérilisés à l'intérieur du conteneur de réception 25.

Dans cette forme de réalisation, la protection contre les radiations des électrons est obtenue par une cellule réalisée en béton, sur le site d'accueil, et à l'intérieur de laquelle vient se loger de façon amovible, le plateau du semi-remorque sur lequel est disposée cette enceinte.

Dans une autre forme de réalisation, une enceinte de radio protection 28, telle que représentée à la figure 2, enveloppe totalement la cuve 17 de l'accélérateur d'électrons 16. Cette enceinte de protection 17 est réalisée à partir de couches concentriques de plomb et/ou d'acier, d'épaisseur adaptée pour éviter toutes les radiations émises par les électrons ainsi que les effets primaires et secondaires qu'elles engendrent.

On conçoit, que ce procédé de traitement de déchets contaminés et ce dispositif pour sa mise en oeuvre se présentant sous une forme compacte, peu consommatrice d'énergie, et susceptible d'être transportée permettent de résoudre les problèmes posés par d'une part le développement des produits dits à usage unique ainsi que celui des maladies infectieuses qui entraîne une augmentation des déchets médicaux dits spécifiques et, d'autre part par le pourcentage de plus en plus important de ces déchets spécifiques qui ne peuvent être stérilisés par un traitement en autoclave ainsi que la limitation de l'utilisation de la stérilisation par un traitement à l'oxyde d'éthylène.

Comme il va de soi, l'invention ne se limite pas à la seule forme de réalisation de ce dispositif pour la mise en oeuvre du procédé de stérilisation de déchets contaminés qui a été décrit ci-dessus, elle en embrasse au contraire, toutes les variantes en respectant le même principe.

C'est ainsi, que l'on ne s'éloignera pas du cadre de l'invention par l'utilisation d'autres moyens de convoyage des déchets, tels qu'une surface support animée d'un mouvement vibratoire, d'autres moyens de contrôle de la régulation, de même que par un réglage adapté de la distance entre le champ du faisceau d'électrons et le transporteur à bande permettant une stérilisation de divers objets destinés à des utilisations variées, telle qu'une stérilisation centrale en milieu hospitalier, par l'utilisation de moyens de nivellement de la couche de déchets constitués par un calibreur à rouleaux ou à vibreur, ou encore par l'utilisation d'un transporteur d'évacuation des déchets hors du transporteur, autre qu'une vis sans fin.

## Revendications

1. Procédé de stérilisation de déchets contaminés, caractérisé en ce qu'il consiste à broyer les déchets contaminés pour obtenir un mélange de granulométrie homogène, à amener le mélange broyé sur un transporteur (12), à niveler le mélange pour obtenir une couche d'épaisseur prédéterminée sur le transporteur et à déplacer le mélange par l'intermédiaire du transporteur à une vitesse déterminée, pour réaliser l'irradiation de la couche de déchets par le champ d'un faisceau d'électrons (15).

2. Procédé de stérilisation selon la revendication 1, caractérisé en ce qu'il consiste à amener le mélange de déchets contaminés à l'extrémité amont d'un transporteur (12) réalisant le déplacement des déchets sous le faisceau d'un champ d'électrons (15) et permettant leur évacuation à partir de son extrémité aval vers l'extérieur.

3. Dispositif pour la mise en oeuvre du procédé selon l'une quelconque des revendications 1 et 2, caractérisé en ce qu'il comprend une enceinte de traitement (3) à l'intérieur de laquelle se trouve un broyeur (7) pour réduire les déchets contaminés en un mélange de granulométrie homogène, un transporteur (12), un moyen de nivellement (13) pour obtenir une couche d'épaisseur régulée du mélange de déchets, et un accélérateur d'électrons (16) pour irradier et stériliser la couche de déchets (3).

4. Dispositif selon la revendication 3, caractérisé en ce que le transporteur (12) comprend un élément sans fin, tel qu'une bande ou une chaîne animée d'un mouvement de déplacement.

5. Dispositif selon la revendication 3, caractérisé en ce que le transporteur comprend une surface support animée d'un mouvement vibratoire destiné à réaliser le déplacement des déchets.

6. Dispositif selon la revendication 4, caractérisé en ce que les moyens de nivellement du mélange de déchets contaminés en une couche d'épaisseur régulière sont constitués par une râcle (13), disposée au-dessus du transporteur, à une distance déterminée de celui-ci, entre la zone d'amenée des déchets et la zone d'irradiation.

7. Dispositif selon la revendication 3, caractérisé en ce que l'accélérateur d'électrons (16) comprend une cuve (17) à l'intérieur de laquelle règne une pression de gaz isolant électrique ainsi qu'un tube accélérateur (26) générant un faisceau d'électrons d'énergie comprise entre 1 et 3 Méga ElectronVolt et un dispositif de balayage (18) comportant au moins un aimant (19) générant un champ magnétique et se terminant par une fenêtre séparant la zone de vide de l'atmosphère.

8. Dispositif selon la revendication 7, caractérisé en ce que l'axe du champ du faisceau d'électrons (15) émis par le dispositif de balayage (18) est perpendiculaire à la direction de déplacement des déchets sur le transporteur (12).

9. Dispositif selon la revendication 7, caractérisé en ce que l'axe du champ d'électrons (15) émis par le dispositif de balayage (18) forme un angle avec la perpendiculaire de la direction de déplacement du transporteur à bande (12).

10. Dispositif selon l'une quelconque des revendications 3 à 9, caractérisé en ce qu'il comprend une trémie étanche (4) de réception des déchets contaminés, en dessous de laquelle est disposé un broyeur réduisant ces déchets en un mélange de granulométrie homogène stocké temporairement à l'intérieur d'un contenant étanche (8) disposé en dessous du broyeur, et dont l'extrémité inférieure comporte un orifice (10) plus ou moins obturable par une trappe (11) disposée au dessous de l'extrémité amont du transporteur (12).

11. Dispositif selon l'une quelconque des revendications 3 à 10, caractérisé en ce que l'extrémité aval du transporteur (12) est disposée au dessus de l'extrémité amont d'un transporteur (20) d'évacuation des déchets stérilés vers l'extérieur.

12. Dispositif selon la revendication 11, caractérisé en ce que le transporteur (20) disposé à l'extrémité aval du transporteur est une vis sans fin montée sur un support articulé et constitué d'éléments modulaires assemblés.

13. Dispositif selon l'une quelconque des revendications 3 à 12, caractérisé en ce qu'il est monté à l'intérieur d'une enceinte (3) équipée de moyens de protection contre les radiations émises par les électrons.

14. Dispositif selon la revendication 13, caractérisé en ce que les moyens de protection sont constitués par une enveloppe (28) de radioprotection entourant la cuve (17) de l'accélérateur d'électrons comprenant au moins une couche de matériaux de protection tels que de l'acier ou du plomb, et par du béton entrant dans la composition des parois de l'enceinte (3).

15. Dispositif selon la revendication 14, caractérisé en ce que dans le cas où la cellule (3) est montée sur un véhicule, elle est destinée, en conditions de traitement des déchets à venir se loger dans une cellule fixe en béton.

## Patentansprüche

1. Verfahren zur Sterilisation von kontaminierten Abfällen, dadurch gekennzeichnet, daß es darin besteht, die kontaminierten Abfälle zu zerkleinern, um eine Mischung mit homogener Granulometrie zu erhalten, die gemahlene Mischung auf eine Transporteinrichtung aufzubringen, die Mischung zu nivellieren, um auf der Transporteinrichtung eine Lage mit vorbestimmter Dicke zu erhalten, und die Lage mit Hilfe der Transporteinrichtung mit einer vorbestimmten Geschwindigkeit zu verlagern, um die Bestrahlung der Lage aus Abfällen durch das Feld einer Elektronenstrahlung auszuführen.

2. Verfahren zur Sterilisation nach Anspruch 1, dadurch gekennzeichnet, daß es darin besteht, die Mischung aus kontaminierten Abfällen auf den stromaufwärtigen Endbereich einer Transporteinrichtung aufzubringen, die das Verlagern der Abfälle unter den Strahl eines Elektronenfeldes (15) ausführt, und deren Abgabe von ihrem stromabwärtigen Endbereich nach außen ermöglicht.

3. Vorrichtung zur Ausführung des Verfahrens nach einem der Ansprüche 1 und 2, dadurch gekennzeichnet, daß sie einen Behandlungsraum (3) enthält, in dessen Innerem sich eine Zerkleinerungseinrichtung (7) zum Überführen der kontaminierten Abfälle in eine Mischung homogener Granulometrie, eine Transporteinrichtung (12), ein Nivelliermittel (13) zum Erhalt einer Lage der Mischung der Abfälle mit gleichmäßiger Dicke und ein Elektronenbeschleuniger (16) zum Bestrahlen und Sterilisieren der Lage aus Abfällen befindet.

4. Vorrichtung nach Anspruch 3, dadurch gekennzeichnet, daß die Transporteinrichtung (12) ein Endloselement wie ein Band oder eine Kette, angetrieben zu einer Verlagerungsbewegung, enthält.

5. Vorrichtung nach Anspruch 3, dadurch gekennzeichnet, daß die Transporteinrichtung eine zum Erhalt der Verlagerung der Abfälle zu einer Vibrationsbewegung angetriebene Tragoberfläche enthält.

6. Vorrichtung nach Anspruch 4, dadurch gekennzeichnet, daß die Mittel zum Nivellieren der Mischung der kontaminierten Abfälle in eine Lage gleichmäßiger Dicke von einem Rakel (13) gebildet werden, das in einem durch die Dicke der Lage bestimmten Abstand oberhalb der Transporteinrichtung zwischen der Zone, in der die Abfälle aufgebracht werden, und der Bestrahlungszone angeordnet ist.

7. Vorrichtung nach Anspruch 3, dadurch gekennzeichnet, daß der Elektronenbeschleuniger (16) ein Gehäuse (17), in dessen Innerem der Druck eines elektrisch isolierenden Gases herrscht, sowie ein Beschleunigungsrohr (26), das einen Elektronenstrahl mit einer Energie zwischen 1 und 3 Megaelektronenvolt erzeugt, und eine Ablenkeinrichtung (18), die mindestens einen ein Magnetfeld erzeugenden Magneten enthält und mit einem die Vakuumzone von der Atmosphäre trennenden Fenster endet, aufweist.

8. Vorrichtung nach Anspruch 7, dadurch gekennzeichnet, daß die Achse des durch die Ablenkeinrichtung (18) abgegebenen Elektronenstrahlfeldes (15) senkrecht zur Richtung der Verlagerung der Abfälle auf der Transporteinrichtung (12) ist.

9. Vorrichtung nach Anspruch 7, dadurch gekennzeichnet, daß die Achse des durch die Ablenkeinrichtung (18) abgegebenen Elektronenfeldes (15) mit der Senkrechten zur Richtung der Verlagerung des Transportbandes (12) einen Winkel bildet.

10. Vorrichtung nach einem der Ansprüche 3 bis 9, dadurch gekennzeichnet, daß sie ein dichtes Füllbehältnis (4) zur Aufnahme der kontaminierten Abfälle aufweist, unterhalb von dem eine Zerkleinerungseinrichtung angeordnet ist, die diese Abfälle in eine Mischung homogener Granulometrie überführt, die vorübergehend im Inneren eines dichten Behälters (8) gespeichert wird, der unterhalb der Zerkleinerungseinrichtung angeordnet ist, und dessen unteres Ende eine Öffnung (10) enthält, die durch eine Verschlußeinrichtung mehr oder weniger verschließbar ist, die oberhalb des stromaufwärtigen Endbereiches der Transporteinrichtung (12) angeordnet ist.

11. Vorrichtung nach einem der Ansprüche 3 bis 10, dadurch gekennzeichnet, daß das stromabwärtige Ende der Transporteinrichtung (12) oberhalb des stromaufwärtigen Endes einer Transporteinrichtung (20) zum Abführen der sterilisierten Abfälle nach außen angeordnet ist.

12. Vorrichtung nach Anspruch 11, dadurch gekennzeichnet, daß die am stromabwärtigen Ende der Transporteinrichtung angeordnete Transporteinrichtung (20) eine an einer gelenkig gelagerten Halteeinrichtung montierte Förderschnecke ist und von zusammengebauten Modulelementen gebildet wird.

13. Vorrichtung nach einem der Ansprüche 3 bis 12, dadurch gekennzeichnet, daß sie im Inneren eines Raums (3) angeordnet ist, der mit Schutzmitteln gegen die durch die Elektronen emittierten Strahlen versehen ist.

14. Vorrichtung nach Anspruch 13, dadurch gekennzeichnet, daß die Schutzmittel durch eine das Gehäuse (17) des Elektronenbeschleunigers umgebende Strahlenschutzhülle (28), die mindestens eine Lage aus Schutzmaterial wie Stahl oder Blei enthält, und durch in den Aufbau der Wände des Raums (3) eingehenden Beton gebildet werden.

15. Vorrichtung nach Anspruch 14, dadurch gekennzeichnet, daß in dem Falle, wenn die Zelle (3) auf einem Fahrzeug montiert ist, diese dazu bestimmt ist, bei der Abfallbehandlung in einer feststehenden Zelle aus Beton untergebracht zu werden.

## Claims

1. Method of sterilising contaminated waste, characterised in that it consists of grinding the contaminated waste in order to obtain a mixture with a homogeneous particle size, feeding the ground mixture onto a conveyor (12), levelling the mixture in order to obtain a layer with a predetermined thickness on the conveyor and moving the mixture by means of the conveyor at a given speed, in order to carry out irradiation of the layer of waste by means of the field of an electron beam (15).

2. Sterilisation method according to Claim 1, characterised in that it consists of feeding the mixture of contaminated waste to the upstream end of a conveyor (12) effecting the movement of the waste underneath the beam of an electron field (15) and enabling it to be discharged from its downstream end to the outside.

3. Device for implementing the method according to either one of Claims 1 or 2, characterised in that it comprises a processing chamber (3) inside which is a grinder (7) for reducing the contaminated waste to a mixture with a homogeneous particle size, a conveyor (12), a levelling means (13) for obtaining a layer of the mixture of waste with a controlled thickness, and an electron accelerator (16) for irradiating and sterilising the layer of waste (3).

4. Device according to Claim 3, characterised in that the conveyor (12) comprises an endless component such as a moving belt or chain.

5. Device according to Claim 3, characterised in that the conveyor comprises a support surface with a vibratory movement designed to move the waste.

6. Device according to Claim 4, characterised in that the means of levelling the mixture of contaminated waste into a layer with an even thickness consist of a scraper (13) disposed above the conveyor at a given distance from the latter, between the area in which the waste is fed in and the irradiation area.

7. Device according to Claim 3, characterised in that the electron accelerator (16) comprises a vessel (17) inside which is an electrically insulating gas pressure and an accelerator tube (26) generating an electron beam with an energy of between 1 and 3 mev and a sweeping device (18) including at least one magnet (19) generating a magnetic field and terminating in a window separating the vacuum zone from the atmosphere.

8. Device according to Claim 7, characterised in that the axis of the field of the electron beam (15) emitted by the sweeping device (18) is perpendicular to the direction of movement of the waste on the conveyor (12).

9. Device according to Claim 7, characterised in that the axis of the electron field (15) emitted by the sweeping device (18) forms an angle with the perpendicular to the direction of movement of the belt conveyor (12).

10. Device according to any one of Claims 3 to 9, characterised in that it comprises a sealed hopper (4) for receiving the contaminated waste, underneath which is disposed a grinder reducing this waste to a mixture with a homogeneous particle size stored temporarily inside a sealed container (8) disposed underneath the grinder, and the bottom end of which has an orifice (10) which can be closed off to a greater or lesser extent by a flap (11) disposed above the upstream end of the conveyor (12).

11. Device according to any one of Claims 3 to 10, characterised in that the downstream end of the conveyor (12) is disposed above the upstream end of a conveyor (20) for discharging the sterile waste to the outside.

12. Device according to Claim 11, characterised in that the conveyor (20) disposed at the downstream end of the conveyor is a worm conveyor mounted on a pivoted support and consisting of modular elements assembled together.

13. Device according to any one of Claims 3 to 12, characterised in that it is mounted inside a chamber (3) equipped with means of protection against the radiation emitted by the electrons.

14. Device according to Claim 13, characterised in that the means of protection consist of a radiation-protection casing (28) surrounding the vessel (17) of the electron accelerator comprising at least one layer of protective material such as steel or lead, and of concrete which is a constituent of the walls of the chamber (3).

15. Device according to Claim 14, characterised in that, where the enclosure (3) is mounted on a vehicle, it is intended, when processing waste, to be housed in a fixed concrete enclosure.
